# EUROPÄISCHE PATENTSCHRIFT

(11) **EP 3 468 663 B1**
(45) Veröffentlichungstag und Bekanntmachung des Hinweises auf die Patenterteilung: **11.03.2020**
(21) Anmeldenummer: 17729115.0
(22) Anmeldetag: 09.06.2017
(51) Int. Cl.: A61N 5/10, A61B 34/30, A61B 5/055, A61B 6/04

(54) **PATIENTENPOSITIONIERVORRICHTUNG UND MEDIZINISCHER ARBEITSPLATZ**
PATIENT-POSITIONING DEVICE AND MEDICAL WORKSTATION
DISPOSITIF DE POSITIONNEMENT DE PATIENT ET POSTE DE TRAVAIL MÉDICAL

(30) Priorität: 14.06.2016 DE 102016210497
(43) Veröffentlichungstag der Anmeldung: 17.04.2019
(73) Patentinhaber: KUKA Deutschland GmbH, 86165 Augsburg (DE)
(72) Erfinder: BENALI, Zoubir, 86199 Augsburg (DE)
(74) Vertreter: Oelke, Jochen
(86) Internationale Anmeldenummer: PCT/EP2017/064184
(87) Internationale Veröffentlichungsnummer: WO 2017/216073

(56) Entgegenhaltungen:
- DE-A1-102005 012 700
- US-A1- 2010 069 920
- US-A1- 2013 025 055
- US-A1- 2015 327 818

## Beschreibung

Die Erfindung betrifft eine Patientenpositioniervorrichtung, die einen Roboterarm und eine Patientenliege umfasst, und einen, die Patientenpositioniervorrichtung umfassenden medizinischen Arbeitsplatz.

Die DE 10 2010 043 421 B4 offenbart einen medizinischen Arbeitsplatz, der eine Vorrichtung zum Erzeugen einer ionisierenden, hochenergetischen Strahlung zum Bestrahlen eines Bereichs eines Lebewesens, eine Lagerungsvorrichtung zum Lagern eines Lebewesens und einen Roboterarm aufweist. Der Roboterarm ist Teil eines Roboters und umfasst eine Befestigungsvorrichtung, an der die Lagerungsvorrichtung befestigt ist.

Die US 2015/327818 A1 lehrt eine Vorrichtung gemäß dem Oberbegriff des Anspruchs 1.

Eine Aufgabe der Erfindung ist es, eine Patientenpositioniervorrichtung mit einem verbesserten Roboterarm anzugeben.

Eine weitere Aufgabe der Erfindung ist es, einen medizinischen Arbeitsplatz mit einer solchen Patientenpositioniervorrichtung anzugeben.

Die Aufgabe der Erfindung wird gelöst durch eine Patientenpositioniervorrichtung, aufweisend eine Patientenliege und eine zum Positionieren der Patientenliege vorgesehenen Roboterarm, der mehrere, hintereinander angeordnete, bezüglich Achsen drehbar gelagerte Glieder umfasst, wobei der Roboterarm als Glieder ein Anfangsglied, ein erstes Glied, ein zweites Glied, ein drittes Glied, ein viertes Glied, ein fünftes Glied und ein sechstes Glied umfasst, wobei das erste Glied im Bereich eines seiner beiden Enden relativ zu einer ersten vertikal verlaufenden Achse bezüglich dem Anfangsglied drehbar gelagert ist, das erste Glied im Bereich seines anderen Endes relativ zu einer zweiten vertikal verlaufenden Achse im Bereich eines der beiden Enden des zweiten Glieds bezüglich dem zweiten Glied drehbar gelagert ist, das zweite Glied im Bereich seines anderen Endes relativ zu einer dritten vertikal verlaufenden Achse im Bereich eines der beiden Enden des dritten Glieds bezüglich dem dritten Glied drehbar gelagert ist, das vierte Glied im Bereich eines seiner Enden relativ zu einer ersten horizontal verlaufenden Achse bezüglich dem dritten Glied im Bereich dessen anderen Endes drehbar gelagert ist, das vierte Glied im Bereich seines anderen Endes relativ zu einer zweiten horizontal verlaufenden Achse im Bereich eines der beiden Enden des fünften Glieds bezüglich dem fünften Glied G5 drehbar gelagert ist, und das fünfte Glied im Bereich seines anderen Endes relativ zu einer dritten horizontal verlaufenden Achse im Bereich eines der beiden Enden des sechsten Glieds bezüglich dem sechsten Glied drehbar gelagert ist, und der Roboterarm die Patientenliege oder eine Befestigungsvorrichtung umfasst, an der die Patientenliege befestigt ist.

Die weitere Aufgabe der Erfindung wird gelöst durch einen medizinischen Arbeitsplatz, aufweisend ein medizintechnisches Gerät und die erfindungsgemäße Patientenpositioniervorrichtung.

Der erfindungsgemäße medizinische Arbeitsplatz ist insbesondere für eine Strahlentherapie vorgesehen. Das medizintechnische Gerät ist somit vorzugsweise eine Vorrichtung zum Erzeugen einer ionisierenden, hochenergetischen Strahlung, wie z.B. Gammastrahlung, Röntgenstrahlung, oder auch beschleunigte Elektronen, Neutronen, Protonen oder schwere Ionen. Die Vorrichtung zum Erzeugen einer ionisierenden, hochenergetischen Strahlung umfasst z.B. eine Strahlungsquelle, welche die ionisierende, hochenergetische Strahlung erzeugt.

Der Roboterarm kann Teil eines Roboters sein, der neben dem Roboterarm eine zum Steuern einer Bewegung des Roboterarms vorgesehene elektronische Steuervorrichtung umfasst. Gemäß einer Variante der erfindungsgemäße Patientenpositioniervorrichtung umfasst diese diesen Roboter.

Der Roboterarm kann Motoren, insbesondere elektrische Motoren, umfassen, die zumindest indirekt angesteuert durch die elektronische Steuervorrichtung eine Bewegung des Roboterarms ermöglichen.

Es kann auch vorgesehen sein, dass der Roboterarm manuell bewegbar ist. Dazu kann z.B. eine mit der elektronischen Steuervorrichtung verbundene Eingabevorrichtung vorgesehen sein, mit der eine Person Eingaben zu tätigen vermag, aufgrund derer die elektronische Steuervorrichtung den Roboterarm den Eingaben entsprechend bewegt. Dadurch werden die Position und Orientierung der Befestigungsvorrichtung und somit der Patientenliege im Raum entsprechend ausgerichtet.

Das medizintechnische Gerät kann eine eigene elektronische Steuervorrichtung aufweisen, welche den bestimmungsgemäßen Betrieb des medizintechnischen Gerätes steuert. Die elektronische Steuervorrichtung des medizintechnischen Gerätes und die elektronische Steuervorrichtung des Roboters können derart eingerichtet sein, dass sie miteinander zu kommunizieren vermögen.

Es kann auch vorgesehen sein, dass der medizinische Arbeitsplatz eine gemeinsame elektronische Steuervorrichtung aufweist, die nicht nur den Roboterarm ansteuert bzw. eingerichtet ist, den Roboterarm anzusteuern, sondern auch den bestimmungsgemäßen Betrieb des medizintechnischen Gerätes steuert, bzw. eingerichtet ist, den bestimmungsgemäß Betrieb des medizintechnischen Gerätes zu steuern.

Der erfindungsgemäße Roboterarm umfasst demnach das Anfangsglied. Vorzugsweise ist es vorgesehen, dass der Roboterarm mit seinem Anfangsglied an einem Boden befestigbar, insbesondere mit dem Boden verschraubbar ist. Vorzugsweise ist der Roboterarm mit seinem Anfangsglied am Boden des medizinischen Arbeitsplatzes befestigt, insbesondere mit diesem Boden verschraubt. Dies erlaubt eine relativ einfache Montage des Roboterarms bzw. der Patientenpositioniervorrichtung.

Das Anfangsglied ist z.B. als eine Bodenplatte ausgebildet, welche vorzugsweise möglichst flach ist.

Der Roboterarm umfasst neben dem Anfangsglied das erste Glied, das zweite Glied und das dritte Glied, die relativ zu den drei vertikal verlaufenden Achsen drehbar gelagert sind. Das erste Glied, das zweite Glied und das dritte Glied erstrecken sich somit in horizontaler Richtung und erlauben eine relativ flexible Ausrichtung der Patientenliege in horizontaler Richtung.

Vorzugsweise ist das dritte Glied über dem zweiten Glied und das zweite Glied über dem ersten Glied angeordnet.

Insbesondere ist das zweite Glied kürzer als das erste Glied und/oder ist das dritte Glied kürzer als das zweite Glied ist.

Dem dritten Glied nachfolgend sind das vierte Glied, das fünfte Glied und das sechste Glied, die bezüglich der horizontal verlaufenden Achsen drehbar gelagert sind. Diese Glieder erlauben somit eine Positionierung der Patientenliege in der Höhe.

Das sechste Glied kann die Befestigungsvorrichtung oder die Patientenliege umfassen, sodass der Roboterarm genau sechs Achsen umfasst.

Gemäß einer bevorzugten Ausführungsform der erfindungsgemäßen Patientenpositioniervorrichtung umfasst der Roboterarm als eines der Glieder ein Endglied, welches die Befestigungsvorrichtung oder die Patientenliege umfasst. In diesem Fall ist das sechste Glied im Bereich eines seiner Enden relativ zur dritten horizontal verlaufenden Achse bezüglich dem fünften Glied drehbar gelagert und das Endglied bezüglich einer siebten Achse des Roboterarms im Bereich des anderen Endes des sechsten Glieds bezüglich dem sechsten Glied drehbar gelagert.

Die siebte Achse verläuft vorzugsweise längs einer Längsachse oder parallel zur Längsachse der Patientenliege. Dadurch ist es möglich, die Patientenliege z.B. um ein paar Grad relativ zu ihrer Längsausdehnung relativ zum Boden zu schwenken. Dies kann z.B. vorteilhaft bei einer Bestrahlung eines auf der Patientenliege liegenden Lebewesens sein.

Die siebte Achse und die dritte horizontal ausgerichtete Achse sind vorzugsweise rechtwinklig zueinander ausgerichtet.

Ein Ausführungsbeispiel der Erfindung ist exemplarisch in den beigefügten schematischen Zeichnungen dargestellt. Es zeigen:
- Figur 1: einen medizinischen Arbeitsplatz mit einer Patientenpositioniervorrichtung und einem medizintechnischen Gerät,
- Figur 2: eine Seitenansicht der Patientenpositioniervorrichtung, und
- Figuren 3 - 6: verschiedene perspektivische Ansichten der Patientenpositioniervorrichtung.

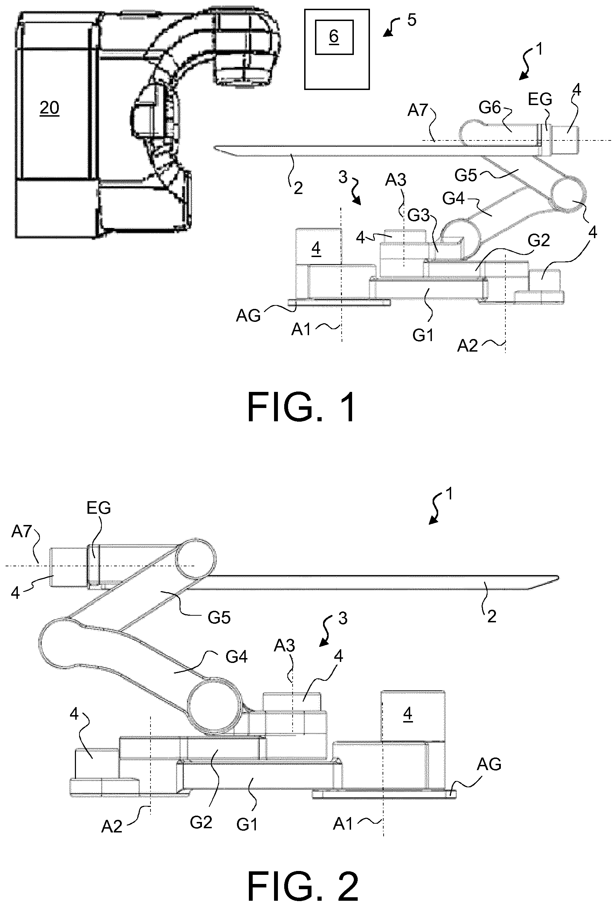

Die Fig. 1 zeigt eine Seitenansicht eines medizinischen Arbeitsplatzes mit einer Patientenpositioniervorrichtung 1 und einem medizintechnischen Gerät 20. Der medizinische Arbeitsplatz ist insbesondere für eine Strahlentherapie vorgesehen.

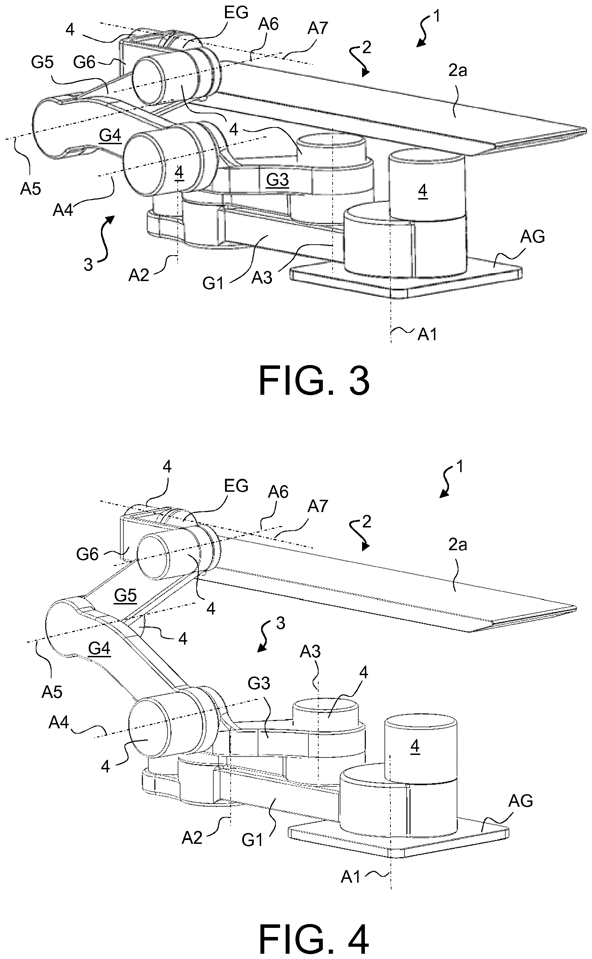

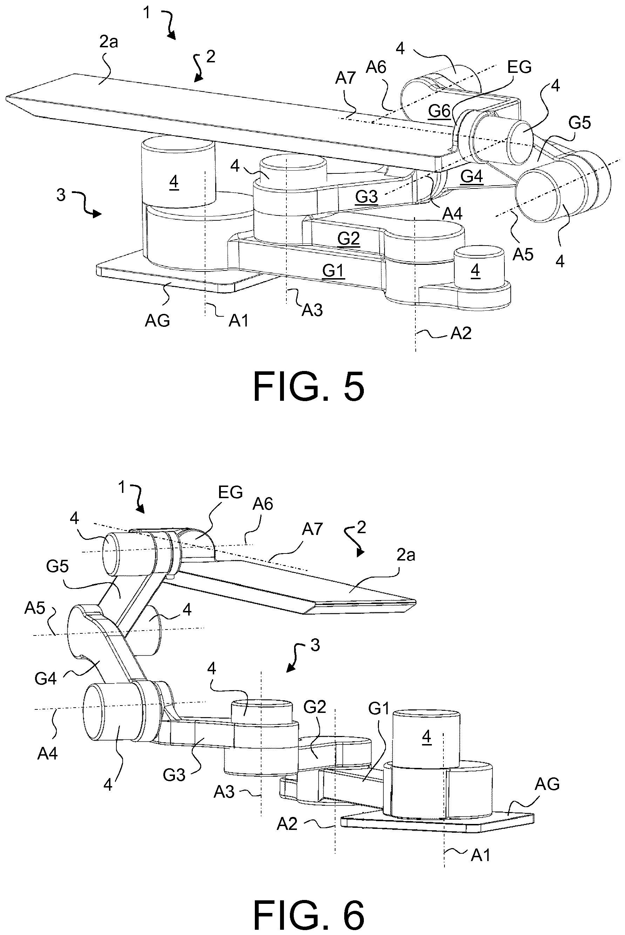

Eine weitere Seitenansicht der Patientenpositioniervorrichtung 1 ist in der Fig. 2 und verschieden perspektivische Ansichten der Patientenpositioniervorrichtung 1 sind in den Figuren 3 bis 6 gezeigt.

Im Falle des vorliegenden Ausführungsbeispiels handelt es sich bei dem medizintechnischen Gerät 20 um eine dem Fachmann im Prinzip bekannte Vorrichtung zum Erzeugen einer ionisierenden, hochenergetischen Strahlung, wie z.B. Gammastrahlung, Röntgenstrahlung, oder auch beschleunigte Elektronen, Neutronen, Protonen oder schwere Ionen. Die Vorrichtung zum Erzeugen einer ionisierenden, hochenergetischen Strahlung umfasst z.B. eine Strahlungsquelle, welche die ionisierende, hochenergetische Strahlung erzeugt.

Die Patientenpositioniervorrichtung 1 umfasst insbesondere eine Patientenliege 2, die eingerichtet ist, dass auf ihr ein nicht dargestelltes Lebewesen zu liegen vermag.

Die Patientenpositioniervorrichtung 1 umfasst einen Roboterarm 3, der mehrere, hintereinander angeordnete Glieder aufweist, die bezüglich Achsen relativ zueinander z.B. mittels Gelenke drehbar gelagert sind. Der Roboterarm 3 stellt eine offene kinematische Kette dar und umfasst somit als eines der Glieder ein Anfangsglied AG und ein Endglied EG. Das Anfangsglied AG ist im Falle des vorliegenden Ausführungsbeispiels eine Bodenplatte, welche am Boden befestigt ist. Das als Bodenplatte ausgeführte Anfangsglied AG ist möglichst flach ausgeführt. Das Anfangsglied AG kann aber auch als ein Sockel ausgeführt sein, mit dem der Roboterarm 3 am Boden befestigt ist.

Das Endglied EG umfasst z.B. eine Befestigungsvorrichtung, an der die Patientenliege 2 befestigt ist, sodass diese mittels des Roboterarms 3 bewegt werden kann. Insbesondere ist die Patientenliege 2 im Bereich einer ihrer Enden an der Befestigungsvorrichtung befestigt.

Im Falle des vorliegenden Ausführungsbeispiels ist es jedoch vorgesehen, dass das Endglied EG die Patientenliege 2 umfasst.

Der Roboterarm 3 umfasst Motoren 4, insbesondere elektrische Motoren, mit denen die einzelnen Glieder des Roboterarms 3 z.B. automatisch bewegt werden können.

Die elektrischen Motoren 4 sind insbesondere jeweils Teil elektrischer Antriebe, deren Stellglieder bzw. Leistungselektroniken z.B. im oder am Roboterarm 3 angeordnet sein können. Im Falle des vorliegenden Ausführungsbeispiels sind die Stellglieder bzw. Leistungselektroniken in einem Steuerschrank 5 angeordnet.

Insbesondere ist der Roboterarm 3 Teil eines Roboters, welcher neben dem Roboterarm 3 eine elektronische Steuervorrichtung 6 aufweist, die z.B. innerhalb des Steuerschranks 5 angeordnet ist.

Die elektronische Steuervorrichtung 6 ist in nicht dargestellter Weise mit den elektrischen Antrieben verbunden.

Auf der elektronischen Steuervorrichtung 6 läuft z.B. ein Rechnerprogramm, sodass die elektronische Steuervorrichtung 6 die Antriebe derart anzusteuern vermag, dass die Position und Orientierung der Befestigungsvorrichtung und somit der Patientenliege 2 im Raum ausgerichtet werden kann. Die elektronische Steuervorrichtung 6 kann gegebenenfalls eingerichtet sein, die Antriebe des Roboters zu regeln, sodass im vorliegenden Fall der Begriff "Steuern" auch den Begriff "Regeln" umfassen soll. Gegebenenfalls sind die elektrischen Antriebe geregelte elektrische Antriebe.

Es kann auch vorgesehen sein, dass der Roboterarm 3 manuell bewegbar ist. Dazu kann z.B. eine mit der elektronischen Steuervorrichtung 6 verbundene, nicht näher dargestellte Eingabevorrichtung vorgesehen sein, mit der eine Person Eingaben zu tätigen vermag, aufgrund derer die elektronische Steuervorrichtung 6 den Roboterarm 3 den Eingaben entsprechend bewegt. Dadurch werden die Position und Orientierung der Befestigungsvorrichtung und somit der Patientenliege 2 im Raum entsprechend ausgerichtet.

Das medizintechnische Gerät 20 kann eine eigene, nicht näher dargestellte elektronische Steuervorrichtung aufweisen, welche den bestimmungsgemäßen Betrieb des medizintechnischen Gerätes 20 steuert. Die elektronische Steuervorrichtung des medizintechnischen Gerätes 20 und die elektronische Steuervorrichtung 6 des Roboters können derart eingerichtet sein, dass sie miteinander zu kommunizieren vermögen.

Es kann auch vorgesehen sein, dass der medizinische Arbeitsplatz eine gemeinsame elektronische Steuervorrichtung aufweist, sodass die elektronische Steuervorrichtung 6 nicht nur den Roboterarm 3 ansteuert, sondern auch den bestimmungsgemäßen Betrieb des medizintechnischen Gerätes 20 steuert.

Im Falle des vorliegenden Ausführungsbeispiels weist der Roboterarm 3 genau sieben Achsen auf.

Der Roboterarm 3 weist genau drei horizontal verlaufende Achsen und drei vertikal verlaufende Achsen auf. Insbesondere umfasst der Roboterarm 3 eine erste vertikal verlaufende Achse A1, eine zweite vertikal verlaufende Achse A2 und eine dritte vertikal verlaufende Achse A3, sowie eine erste horizontal verlaufende Achse A4, eine zweite horizontal verlaufende Achse A5 und eine dritte horizontal verlaufende Achse A6.

Als Glieder umfasst der Roboterarm 3 insbesondere ein erstes Glied G1, ein zweites Glied G2, ein drittes Glied G3, ein viertes Glied G4, ein fünftes Glied G5 und ein sechstes Glied G6, die zwischen dem Anfangsglied AG und dem Endglied EG angeordnet sind.

Das erste Glied G1 ist im Bereich eines seiner beiden Enden relativ zur ersten vertikal verlaufenden Achse A1 bezüglich dem Anfangsglied AG drehbar gelagert.

Das erste Glied G1 ist im Bereich seines anderen Endes relativ zur zweiten vertikal verlaufenden Achse A2 im Bereich eines der beiden Enden des zweiten Glieds G2 bezüglich dem zweiten Glied G2 drehbar gelagert.

Das zweite Glied G2 ist im Bereich seines anderen Endes relativ zur dritten vertikal verlaufenden Achse A3 im Bereich eines der beiden Enden des dritten Glieds G3 bezüglich dem dritten Glied G3 drehbar gelagert.

Das vierte Glied G4 ist im Bereich eines seiner Enden relativ zur ersten horizontal verlaufenden Achse A4 bezüglich dem dritten Glied G3 im Bereich dessen anderen Endes drehbar gelagert.

Das vierte Glied G4 ist im Bereich seines anderen Endes relativ zur zweiten horizontal verlaufenden Achse A5 im Bereich eines der beiden Enden des fünften Glieds G5 bezüglich dem fünften Glied G5 drehbar gelagert.

Das fünfte Glied G5 ist im Bereich seines anderen Endes relativ zur dritten horizontal verlaufenden Achse A6 im Bereich eines der beiden Enden des sechsten Glieds G6 bezüglich dem sechsten Glied G6 drehbar gelagert.

Im Bereich des anderen Endes des sechsten Glieds G6 ist das Endglied EG bezüglich einer siebten Achse A7 des Roboterarms drehbar gelagert.

Die siebte Achse A7 ist derart ausgerichtet, dass sie längs der Längsachse bzw. parallel zur Längsachse der Patientenliege 2 verläuft. Dadurch ist es möglich, die Patientenliege um ein paar Grad zu schwenken.

Vorzugsweise sind die siebte Achse und die sechste Achse A6 rechtwinklig zueinander ausgerichtet. Vorzugsweise schneiden sich die siebte Achse A7 und die sechste Achse A6.

Das erste Glied G1, das zweite Glied G2 und das dritte Glied G3 erstrecken sich somit in horizontaler Richtung.

Insbesondere ist das dritte Glied G3 über dem zweiten Glied G2 und das zweite Glied G2 über dem ersten Glied G1 angeordnet. Das dritte Glied G3 ist vorzugsweise kürzer als das zweite Glied G2.

Es ist auch möglich, dass der Roboterarm 3 nicht die siebte Achse 7 und das Endglied EG umfasst, sondern dass das sechste Glied G6 die Patientenliege 2 umfasst oder dass das sechste Glied G6 die Befestigungsvorrichtung zum Befestigen der Patientenliege 2 umfasst.

## Patentansprüche

1. Patientenpositioniervorrichtung, aufweisend eine Patientenliege (2) und einen zum Positionieren der Patientenliege (2) vorgesehenen Roboterarm (3), der mehrere, hintereinander angeordnete, bezüglich Achsen drehbar gelagerte Glieder umfasst, wobei der Roboterarm (3) als Glieder ein Anfangsglied (AG), ein erstes Glied (G1), ein zweites Glied (G2), ein drittes Glied (G3), ein viertes Glied (G4), ein fünftes Glied (G5) und ein sechstes Glied (G6) umfasst, **dadurch gekennzeichnet, dass** das erste Glied (G1) im Bereich eines seiner beiden Enden relativ zu einer ersten vertikal verlaufenden Achse (A1) bezüglich dem Anfangsglied (AG) drehbar gelagert ist, das erste Glied (G1) im Bereich seines anderen Endes relativ zu einer zweiten vertikal verlaufenden Achse (A2) im Bereich eines der beiden Enden des zweiten Glieds (G2) bezüglich dem zweiten Glied (G2) drehbar gelagert ist, das zweite Glied (G2) im Bereich seines anderen Endes relativ zu einer dritten vertikal verlaufenden Achse (A3) im Bereich eines der beiden Enden des dritten Glieds (G3) bezüglich dem dritten Glied (G3) drehbar gelagert ist, das vierte Glied (G4) im Bereich eines seiner Enden relativ zu einer ersten horizontal verlaufenden Achse (A4) bezüglich dem dritten Glied (G3) im Bereich dessen anderen Endes drehbar gelagert ist, das vierte Glied (G4) im Bereich seines anderen Endes relativ zu einer zweiten horizontal verlaufenden Achse (A5) im Bereich eines der beiden Enden des fünften Glieds (G5) bezüglich dem fünften Glied G5 drehbar gelagert, und das fünfte Glied (G5) im Bereich seines anderen Endes relativ zu einer dritten horizontal verlaufenden Achse (A6) bezüglich dem sechsten Glied (G6) drehbar gelagert ist, und der Roboterarm (3) die Patientenliege (2) oder eine Befestigungsvorrichtung umfasst, an der die Patientenliege (2) befestigt ist.

2. Patientenpositioniervorrichtung nach Anspruch 1, **dadurch gekennzeichnet, dass** das Anfangsglied (AG) an einem Boden befestigbar, insbesondere mit dem Boden verschraubbar ist.

3. Patientenpositioniervorrichtung nach Anspruch 1 oder 2, **dadurch gekennzeichnet, dass** das dritte Glied (G3) über dem zweiten Glied (G2) und das zweite Glied (G2) über dem ersten Glied (G1) angeordnet ist.

4. Patientenpositioniervorrichtung nach einem der Ansprüche 1 bis 3, **dadurch gekennzeichnet, dass** das zweite Glied (G2) kürzer als das erste Gliede (G1) ist, und/oder dass das dritte Glied (G3) kürzer als das zweite Gliede (G2) ist.

5. Patientenpositioniervorrichtung nach einem der Ansprüche 1 bis 4, **dadurch gekennzeichnet, dass** das sechste Glied (G6) die Befestigungsvorrichtung oder die Patientenliege (2) umfasst.

6. Patientenpositioniervorrichtung nach einem der Ansprüche 1 bis 4, **dadurch gekennzeichnet, dass** der Roboterarm (3) als eines der Glieder ein Endglied (EG) aufweist, das bezüglich einer siebten Achse (A7) des Roboterarms (3) relativ zum sechsten Glied (G6) drehbar gelagert ist und die Befestigungsvorrichtung oder die Patientenliege (2) umfasst.

7. Patientenpositioniervorrichtung nach Anspruch 6, **dadurch gekennzeichnet, dass** die siebte Achse (A7) längs einer Längsachse oder parallel zur Längsachse der Patientenliege (2) verläuft und/oder dass die siebte Achse (A7) und die dritte horizontale Achse (AG) rechtwinklig zueinander ausgerichtet sind.

8. Patientenpositioniervorrichtung nach einem der Ansprüche 1 bis 7, **gekennzeichnet durch** einen Roboter, der den Roboterarm (3) und eine elektronische Steuervorrichtung (6) umfasst, die eingerichtet ist, eine Bewegung des Roboterarms (3) zu steuern.

9. Medizinischer Arbeitsplatz, aufweisend ein medizintechnisches Gerät (20) und die Patientenpositioniervorrichtung nach einem der Ansprüche 1 bis 8.

## Claims

1. Patient positioning device, comprising a patient couch (2) and a robot arm (3) provided for positioning the patient couch (2), which comprises a plurality of links arranged one behind the other, rotatably mounted with respect to axes, the robot arm (3) as links an initial link (AG), comprises a first link (GI), a second link (G2), a third link (G3), a fourth link (G4), a fifth link (G5) and a sixth link (G6), **characterized in that** the first Link (GI) is rotatably mounted in the area of one of its two ends relative to a first vertical axis (AI) with respect to the starting member (AG), the first link (GI) in the area of its other end relative to a second vertical axis (A2) is rotatably mounted in the area of one of the two ends of the second link (G2) with respect to the second link (G2), the second link (G2) in the area of its other end relative to a third vertically extending one Axis (A3) is rotatably mounted in the area of one of the two ends of the third link (G3) with respect to the third link (G3), the fourth link (G4) in the area of one of its ends relative to a first horizontally extending axis (A4) with respect to the third link (G3) is rotatably mounted in the area of its other end, the fourth link (G4) in the area of its other end relative to a second horizontal axis (A5) in the area of one of the two ends of the fifth link (G5) with respect to the fifth Link G5 is rotatably mounted, and the fifth link (G5) is rotatably mounted in the region of its other end relative to a third horizontally extending axis (A6) with respect to the sixth link (G6), and the robot arm (3) the patient couch (2) or comprises a fastening device to which the patient bed (2) is fastened.

2. Patient positioning device according to claim 1, **characterized in that** the starting member (AG) can be fastened to a floor, in particular screwed to the floor.

3. Patient positioning device according to claim 1 or 2, **characterized in that** the third link (G3) is arranged above the second link (G2) and the second link (G2) above the first link (Gl).

4. Patient positioning device according to one of claims 1 to 3, **characterized in that** the second link (G2) is shorter than the first link (GI) and / or that the third link (G3) is shorter than the second link (G2).

5. Patient positioning device according to one of claims 1 to 4, **characterized in that** the sixth link (G6) comprises the fastening device or the patient couch (2).

6. Patient positioning device according to one of claims 1 to 4, **characterized in that** the robot arm (3) as one of the links has an end link (EG) which is rotatable relative to the sixth link (G6) with respect to a seventh axis (A7) of the robot arm (3) is mounted and includes the fastening device or the patient couch (2).

7. Patient positioning device according to claim 6, **characterized in that** the seventh axis (A7) runs along a longitudinal axis or parallel to the longitudinal axis of the patient couch (2) and / or that the seventh axis (A7) and the third horizontal axis (AG) are perpendicular to each other are aligned.

8. Patient positioning device according to one of claims 1 to 7, **characterized by** a robot which comprises the robot arm (3) and an electronic control device (6) which is set up to control a movement of the robot arm (3).

9. Medical work station, comprising a medical device (20) and the patient positioning device according to one of claims 1 to 8.

## Revendications

1. Dispositif de positionnement de patient, comprenant un divan de patient (2) et un bras de robot (3) prévu pour positionner le divan de patient (2), qui comprend une pluralité de maillons disposés les uns derrière les autres, montés rotatifs par rapport à des axes, le bras de robot (3) faisant le lien avec un maillon initial (AG), comprend une première liaison (GI), une deuxième liaison (G2), une troisième liaison (G3), une quatrième liaison (G4), une cinquième liaison (G5) et une sixième liaison (G6), **caractérisées en ce que** la première La liaison (GI) est montée rotative dans la zone de l'une de ses deux extrémités par rapport à un premier axe vertical (AI) par rapport à l'élément de départ (AG), la première liaison (GI) dans la zone de son autre extrémité par rapport à un deuxième axe vertical (A2) est monté rotatif dans la zone de l'une des deux extrémités de la deuxième liaison (G2) par rapport à la deuxième liaison (G2), la deuxième liaison (G2) dans la zone de son autre extrémité par rapport à une troisième s'étendant verticalement L'axe (A3) dans la zone de l'une des deux extrémités de la troisième liaison (G3) par rapport à la troisième liaison (G3) est monté rotatif, la quatrième liaison (G4) dans la zone de l'une de ses extrémités par rapport à un premier axe s'étendant horizontalement (A4) par rapport à la le troisième maillon (G3) est supporté en rotation dans la zone de son autre extrémité, le quatrième maillon (G4) dans la zone de son autre extrémité par rapport à un deuxième axe s'étendant horizontalement (A5) dans la zone de l'une des deux extrémités du cinquième maillon (G5) par rapport à la cinquième La liaison G5 est montée rotative, et la cinquième liaison (G5) est montée rotative dans la région de son autre extrémité par rapport à un troisième axe s'étendant horizontalement (A6) par rapport à la sixième liaison (G6), et le bras du robot (3) le lit patient (2) ou comprend un dispositif de fixation auquel le lit de patient (2) est fixé.

2. Dispositif de positionnement de patient selon la revendication 1, **caractérisé en ce que** l'organe de départ (AG) peut être fixé à un sol, notamment vissé au sol.

3. Dispositif de positionnement de patient selon la revendication 1 ou 2, **caractérisé en ce que** le troisième lien (G3) est disposé au-dessus du deuxième lien (G2) et le deuxième lien (G2) au-dessus du premier lien (GI).

4. Dispositif de positionnement de patient selon l'une des revendications 1 à 3, **caractérisé en ce que** le deuxième lien (G2) est plus court que le premier lien (GI), et / ou que le troisième lien (G3) est plus court que le deuxième lien (G2).

5. Dispositif de positionnement de patient selon l'une des revendications 1 à 4, **caractérisé en ce que** le sixième maillon (G6) comprend le dispositif de fixation ou le lit de patient (2).

6. Dispositif de positionnement de patient selon l'une des revendications 1 à 4, **caractérisé en ce que** le bras de robot (3) comme l'un des maillons a un maillon d'extrémité (EG) qui peut tourner par rapport au sixième maillon (G6) par rapport à un septième axe (A7) du bras de robot (3) est monté et comprend le dispositif de fixation ou le canapé patient (2).

7. Dispositif de positionnement de patient selon la revendication 6, **caractérisé en ce que** le septième axe (A7) s'étend le long d'un axe longitudinal ou parallèle à l'axe longitudinal du canapé patient (2) et / ou que le septième axe (A7) et le troisième axe horizontal (AG) sont perpendiculaires l'un à l'autre sont alignés.

8. Dispositif de positionnement de patient selon l'une des revendications 1 à 7, **caractérisé par** un robot qui comprend le bras de robot (3) et un dispositif de commande électronique (6) qui est configuré pour commander un mouvement du bras de robot (3).

9. Poste de travail médical, comprenant un dispositif médical (20) et le dispositif de positionnement de patient selon l'une des revendications 1 à 8.
